# EUROPÄISCHE PATENTSCHRIFT

(11) **EP 3 500 137 B1**
(45) Veröffentlichungstag und Bekanntmachung des Hinweises auf die Patenterteilung: **09.12.2020**
(21) Anmeldenummer: 17742770.5
(22) Anmeldetag: 27.07.2017
(51) Int. Cl.: A47C 1/034, A47C 1/0355

(54) **SITZMÖBELSTÜCK**
ITEM OF SEATING FURNITURE
ELEMENT DE SIEGE

(30) Priorität: 16.08.2016 EP 16184390
(43) Veröffentlichungstag der Anmeldung: 26.06.2019
(73) Patentinhaber: Beheermaatschappij Vermeulen Beesd B.V., 4104 BA Culemborg (NL)
(72) Erfinder: FISCHER, Matthias, 81101 Bratislava (SK)
(74) Vertreter: Patentanwaltskanzlei Cartagena
(86) Internationale Anmeldenummer: PCT/EP2017/069058
(87) Internationale Veröffentlichungsnummer: WO 2018/033362

(56) Entgegenhaltungen:
- EP-A1- 2 777 433
- DE-U1-202015 105 292
- US-A1- 2012 248 831

## Beschreibung

### ANWENDUNGSGEBIET UND STAND DER TECHNIK

Die Erfindung betrifft ein Sitzmöbelstück, insbesondere in Form eines Sessels, sowie einen Beschlag hierfür.

Gattungsgemäße und erfindungsgemäße Sitzmöbelstücke verfügen über eine Basis zur ortsfesten Aufstellung, über ein darüber und demgegenüber verkippbar angeordnetes Sitzelement mit Sitzfläche sowie ein per Laschengetriebe am Sitzelement angebrachtes Beinauflageelement mit einer Beinauflagefläche. Das Laschengetriebe gestattet es, durch eine um eine Möbelquerachse verschwenkbare Lasche das Beinauflageelement zwischen einer Staustellung unter dem Sitzelement und einer Nutzstellung vor dem Sitzelementzu bewegen.

Aus der EP 2609834 A1 ist ein gattungsgemäßes Möbelstück bekannt, bei dem ein gegenüber einem Sitzelement verlagerbarer Zwischenträger über das Laschengetriebe angetrieben ist. An diesem Zwischenträger ist eine Teleskopstange angebracht, an deren gegenüber dem Zwischenträger verschieblichem Ende eine Beineinheit vorgesehen ist. Das Laschengetriebe zur Verlagerung des Zwischenträgers ist außenseitig der Sitzfläche vorgesehen.

Aus der EP 2084992 A2 ist ein anderes Sitzmöbelstück bekannt, bei dem eine mehrgliederige Beinauflage in kettenartiger Anbindung an einem Sitzelement vorgesehen ist. Die einzelnen Elemente werden dabei nacheinander ausgeschwenkt, wobei auch bereits die motorisierte Gestaltung vorgeschlagen wird. Aus der US2012/248831 A1 ist ein weiteres Sitzmöbelstück bekannt, welches dem der vorliegenden Erfindung ähnlich ist.

Auch andere Sitzmöbelstücke in Form von Sesseln mit elektrischem Antrieb zurVerlagerung von Elementen des Sitzmöbelstücks zueinander sind bekannt. Meist ist jedoch der Bauraum zum Anbringen solcher Motoren beschränkt und macht es meist erforderlich, ein Sitzmöbelstück von vornherein als elektrisches Sitzmöbelstück zu entwerfen, um den erforderlichen Bauraum von Anfang an einplanen zu können. Zudem haben elektrische Sitzmöbelstücke auch das Problem, dass eine vergleichsweise hohe Verletzungsgefahr besteht, wenn die Einzelelemente und Getriebeteile motorisch gegeneinander bewegt werden, insbesondere da die Getriebe elektrisch angetriebener Sitzmöbelstücke häufig so groß ausgebildet sind, dass sie bis zum Teil die Außenkonturen des Sitzmöbelstücks bilden.

### AUFGABE UND LÖSUNG

Aufgabe der Erfindung ist es, ein Sitzmöbelstück und einen hierfür vorgesehenen Beschlag zur Verfügung zu stellen, die elektrisch betrieben werden können und eine flexible Bauweise in Hinblick auf die Anbringung an Sitzmöbeln verschiedenen Typs gestatten.

Erfindungsgemäß wird hierfür ein Sitzmöbelstück gemäß Anspruch 1 vorgeschlagen, das über eine Basis zur ortsfesten Aufstellung und über ein gegenüber der Basis kippbewegliches Sitzelement mit einer Sitzfläche und einer Tragstruktur verfügt.

Die Basis kann als Ganzes starr und zur ortsfesten Aufstellung ausgebildet sein oder als zweiteilige Basis mit einem ortsfest aufzustellenden Fuß und einem demgegenüber um eine vertikale Drehachse drehbarer Basisabschnitt ausgebildet sein. Die Basis bzw. der drehbare Basisabschnitt sind zur kippbeweglichen Abbringung des Sitzelements vorgesehen. Eine besondere Form einer Basis sieht vor, dass zwischen deren Aufstellfläche und der dem Basisabschnitt, an dem das Sitzelement angebracht ist, eine so genannte Aufstehhilfe vorgesehen ist, also ein Beschlagsabschnitt, mittels dessen das Sitzelement und alle daran vorgesehenen weiteren Elemente gemeinsam angehoben und leicht nach vorne geneigt werden können, um das Aufstehen vom Sitzmöbelstück zu erleichtern.

Das Sitzmöbelstück verfügt weiterhin über ein gegenüber dem Sitzelement bewegliches Beinauflagenelement mit einer Beinauflagefläche und einer Tragstruktur, welches mittels eines Laschengetriebes mit dem Sitzelement verbunden ist, wobei mittels des Laschengetriebes das Beinauflageelement zwischen einer Staustellung unter dem Sitzelement und einer Nutzstellung vor dem Sitzelement beweglich ist. Ein Laschengetriebe weist mindestens eine langestreckte Schwenklasche auf, welche das Hauptelement für die Verlagerung des Beinauflageelements in Möbellängsrichtung ist. Die mindestens eine Lasche ist beidseitig schwenkbar angebracht, nämlich um eine in Möbelquerrichtung erstreckte Achse an einem dem Sitzelement zugeordneten Anbringungsabschnitt und ebenfalls um eine in Möbelquerrichtung erstreckte Achse an einem dem Beinauflageelement zugeordneten Anbringungsabschnitt. Die langgestreckte Lasche verschwenkt vorzugsweise um einen Winkel von mehr als 60°, so dass die beinauflagenelementseitige Schwenkachse mindestens um die Länge der Lasche bzw. den Abstand der Schwenkachsen der Lasche verlagert wird.

Als Antrieb des Laschengetriebes ist ein Elektromotor am erfindungsgemäßen Sitzmöbelstück unterhalb der Sitzfläche vorgesehen. Dieser wirkt über eine Schubstange auf das Laschengetriebe, um hierdurch die mindestens eine Schwenklasche in Richtung der Nutzstellung des Beinauflageelements anzutreiben. Es kann sich bei der Schubstange um den Läufer eines Linearaktuators handeln. Vorzugsweise jedoch ist auch bei der Verwendung eines Linearaktuators eine vom Läufer getrennte Schubstange vorgesehen, dessen Bewegungsablauf sich von dem des Läufers unterscheidet.

Das Beinauflageelement und die die Oberseite des Beinauflageelements bildende Beinauflagefläche sind bei der erfindungsgemäßen Gestaltung geschlitzt ausgebildet. Es ist also eine zur Sitzfläche in etwa zentrierte Anordnung mit mindestens einem Schlitz oder mehreren nah beieinander angeordneten Schlitzen mit Gesamtbreite der Schlitzanordnung < 20 cm vorgesehen, die es durch Eintauchen der mindestens einen Schwenklasche erlauben, dass das Beinauflageelement in der Staustellung unterhalb der sitzelementseitigen Schwenkachse der Schwenklasche angeordnet ist. Das Beinauflageelement kann hierdurch zwischen seiner Staustellung unter dem Sitzelement und seiner Nutzstellung vor dem Sitzelement gleichorientiert bleiben, also derart, dass die Beinauflagefläche zumindest in der Staustellung als auch der Nutzstellung, vorzugsweise jedoch auch in jeder Zwischenstellung, in etwa horizontal mit nach oben weisender Beinauflagefläche ausgerichtet ist (+/- 45°). Diese Art der schwenkarmen Verlagerung des Beinauflageelements führt zu einem vergleichsweise kleinen Bewegungsraum, der für die Überführung benötigt wird. Verglichen mit Sitzmöbelstücken, bei denen das Beinauflageelement am Sitzflächenelement angelenkt ist und somit eine reine Schwenkbewegung, üblicherweise von 75° oder mehr, zum Zwecke des Ausfahrens erfolgt, kann die maximale Länge des Beinauflageelements bei der erfindungsgemäßen Gestaltung erheblich größer sein.

Die zentrierte Anordnung der mindestens einen Schwenklasche führt dazu, dass die Gefahr eines Einklemmens beispielsweise eines Fingers oder eines Körperteils eines Haustieres aufgrund der fehlenden Zugänglichkeit sehr unwahrscheinlich ist. Zudem kann ein solches Sitzmöbelstück über einen die Kinematik der Elemente definierenden Beschlag verfügen, der nicht an die konkrete Breite eines konkreten Sitzmöbelstücks angepasst werden muss und der aufgrund der in etwa zentrischen Anordnung der Schwenklasche auch eine in etwa zentrische oder nur leicht exzentrische Anordnung des Motors gestattet, so dass dieser von außen schwer zu erkennen ist. Dies gestattet es, den Beschlag ohne weitere Anpassungen oder mit nur geringen Anpassungen für bestehende Typen von Sitzmöbelstücken zu verwenden und diese mit einem ausfahrbaren Beinauflageelement zu versehen. Insbesondere können sogar Sessel ohne Seitenteilen beidseitig des Sitzelements vorgesehen sein, da der Beschlag derart klein gebaut sein kann, dass er selbst bei einer solchen Gestaltung kaum sichtbar ist.

Die zentrierte Anordnung der Schwenklasche und die ebenfalls vorzugsweise gegebene zentrierte Anordnung aller anderen Getriebekomponenten hat sich darüber hinaus als sehr vorteilhaft herausgestellt, da es kaum Probleme mit unter Last sich biegenden Komponenten gibt, wie es zu befürchten ist, wenn das Getriebe zentrische und deutlich exzentrische Teilkomponenten aufweist.

Die Tragstruktur des Sitzelements weist vorzugsweise zwei zueinander parallele Wangen auf, wobei diese insbesondere vorzugsweise über einen Zapfen miteinander verbunden sind oder von diesem durchdrungen sind, der die Hauptachse zum Verkippen des Sitzelements gegenüber der Basis definiert. Diese Wangen stehen nah beieinander und sind vorzugsweise um nicht mehr als 15 cm voneinander beabstandet. Der genannte Elektromotor kann in einem Zwischenraum zwischen diesen Wangen angeordnet sein. Als vorteilhaft hat sich jedoch eine Gestaltung herausgestellt, bei der der Elektromotor exzentrisch und außerhalb des durch die Wangen gebildeten Zwischenraums angeordnet ist. Bei einer solchen Gestaltung ist es von Vorteil, wenn der Elektromotor auf ein an den Wangen drehbar gelagertes Steuerglied wirkt, welches sich durch den Zwischenraum zwischen den Wangen auf die in Möbelquerrichtung gegenüberliegende Seite erstreckt, wo es mit dem Kippgetriebe und/oder dem Laschengetriebe wirkverbunden ist. Auch die Tragstruktur des Beinauflageelements kann ebenso zwei nah beieinander angeordnete Wangen aufweisen, wobei diese zwischen sich den genannten Schlitz definieren können. Die mindestens eine Schwenklasche ist vorzugsweise auf Seiten des Beinauflageelements und auf Seiten des Sitzelements zwischen den jeweiligen Wangen gelagert.

Dabei wird es als besonders vorteilhaft angesehen, wenn die Schwenklaschen nicht unmittelbar benachbart zu den Wangen angeordnet sind, sondern in Möbelquerrichtung um vorzugweise mindestens 10 mm, insbesondere vorzugsweise mindestens 15 mm beabstandet sind. Auf diese Weise wird hier einen Scherenwirkung unterbunden und die Verletzungsgefahr für Kinder und Haustiere gesenkt.

Bei einem erfindungsgemäßen Sitzmöbelstück ist vorgesehen, dass sowohl das Sitzelement gegenüber der Basis als auch das Beinauflageelement gegenüber dem Sitzelement beweglich ist. Dies kann über zwei getrennte Elektromotoren erreicht werden. Vorzugsweise ist jedoch zur Steuerung einer Kippbewegung des Sitzelements ein Kippgetriebe vorgesehen, welches mittels des gleichen Elektromotors angetrieben werden kann, der auch als Antrieb des Laschengetriebes zur Verlagerung des Beinelementes dient. Der Elektromotor wirkt bei einer solchen Gestaltung mittels eines gemeinsamen Steuergliedes einerseits auf das Kippgetriebe und andererseits auf das Laschengetriebe.

Bei einem erfindungsgemäßen Sitzmöbelstück ist vorgesehen, dass es mindestens zwei, vorzugsweise aber drei unterschiedliche Konfigurationen vorsieht, nämlich eine Sitzkonfiguration, bei der die Beinauflagefläche nicht genutzt wird und unterhalb der Sitzfläche angeordnet ist, sowie eine oder zwei Liegekonfigurationen, in denen das Beinauflageelement ausgefahren und in Nutzstellung ist.

Wenn zwei Liegekonfigurationen vorgesehen sind, dann handelt es sich vorzugsweise um eine Fernsehposition und eine Schlafposition, welche sich primär dadurch unterscheiden, dass das Sitzelement in unterschiedlichem Maße gegenüber der Basis verschwenkt ist, wobei vorzugsweise die Nutzstellung des Beinauflageelements relativ zum Sitzelement in der Fernsehposition und der Schlafposition identisch ist.

Insbesondere, wenn ein gemeinsamer Motor für das Verkippen des Sitzelements und das Ausfahren des Beinauflageelements vorgesehen ist, ist es sinnvoll, das Kippgetriebe und/oder das Laschengetriebe in besonderer Art auszugestalten, um die beiden Bewegungen zeitlich aufeinander abzustimmen.

Das Kippgetriebe kann daher dafür ausgebildet sein, bei der Bewegung des gemeinsamen Steuergliedes aus einer Anfangsstellung in eine Endstellung eine Verlagerung des Sitzelements gegenüber der Basis zu bewirken, wobei in mindestens einer Bewegungsphase des Steuergliedes die Relativstellung des Sitzelements zur Basis unverändert bleibt.

Alternativ oder zusätzlich kann das Laschengetriebe dafür ausgebildet sein, bei der Bewegung des gemeinsamen Steuergliedes aus einer Anfangsstellung in eine Endstellung eine Verlagerung des Beinauflageelements gegenüber dem Sitzelement zu bewirken, wobei das Laschengetriebe weiterhin derart ausgebildet ist, dass in mindestens einer Bewegungsphase des Steuergliedes die Relativstellung des Beinauflageelements zum Sitzelement unverändert bleibt.

Unter einer Bewegungsphase im Sinne dieser zeitweise entkoppelten Wirkkopplungen wird eine Phase von mindestens 10% einer Wegstrecke des gemeinsamen Steuergliedes aus der Anfangsstellung in die Endstellung verstanden. Vorzugsweise ist die Phase jeweils länger vorgesehen, insbesondere 20% der Wegstrecke umfassend. Unter der Unveränderlichkeit der Relativstellung des Beinauflageelements zum Sitzelement bzw. des Sitzelements gegenüber der Basis ist zu verstehen, dass das jeweilige Element bezogen auf seine spezifische eigene Gesamtverlagerung gegenüber seinem Bezugselement bei der Überführung aus der Sitzkonfiguration in die Liegekonfigurationen nur vernachlässigbar (<3%) in einer solchen Phase verlagert wird.

Zur technischen Realisierung der genannten Entkoppelung sind Kulissenführungen gut geeignet, wie im Weiteren noch erläutert wird.

Die Abstimmung der Relativbewegungen erfolgt vorzugsweise wie folgt: In einer ersten Bewegungsphase wird das Beinauflageelement aus der Staustellung in die Nutzstellung überführt, so dass damit bereits die Fernsehkonfiguration erzielt ist. In dieser Bewegungsphase erfolgt die Verlagerung primär über das Laschengetriebe. Allerdings kann es zweckmäßig sein, dass zu Beginn dieser Phase auch ein Verkippen über das Kippgetriebe erfolgt, um den Platz unter dem Sitzelement hierdurch zu vergrößern. Hierdurch kann der erforderliche Platz unter dem Sitzelement geschaffen werden, entlang dessen das Laschengetriebe das Beinauflageelement ausfahren kann und/oder es kann bewirkt werden, dass ein ausreichender Abstand zwischen der Unterseite des Beinauflageelements und dem Fußboden beim Ausfahren verbleibt, so dass keine Gefahr für Kinderhände oder Haustierpfoten besteht.

In einer sich daran anschließenden zweiten Bewegungsphase wird das Sitzelement gemeinsam mit dem Beinauflageelement gegenüber der Basis verkippt, während das Beinauflageelement ortsfest zum Sitzelement verbleibt, seine Relativstellung zum Sitzelement also nicht oder nur vernachlässigbar verändert. Diese zweite Bewegungsphase endet in der Liegeposition, in der das Sitzelement maximal verkippt ist und das Beinauflageelement seine höchste Stellung einnimmt.

Dadurch, dass in der zweiten Bewegungsphase keine nennenswerte Verlagerung mehr zwischen der Beinauflage und dem Sitzelement erfolgt, wird ermöglicht, dass die Sitzfläche und die Beinauflagefläche in der Fernsehkonfiguration und der Schlafkonfiguration gut zueinander fluchten. Würde auch in dieser Bewegungsphase noch eine nennenswerte Relativverlagerung zwischen den beiden Elementen stattfinden, so wären aufgrund des Laschengetriebes die Flächen in mindestens einer Konfiguration nicht fluchtend ausgerichtet, sondern würden eine Art Stufe bilden.

Damit die Ausrichtung der Beinauflage in der Schlafstellung als angenehm empfunden wird, ist es von Vorteil, wenn die Beinauflage in dieser Stellung horizontal oder leicht nach hinten hin abfällt, also in Richtung der Sitzfläche geneigt ist. Damit dies bei in der zweiten Bewegungsphase in unveränderter Nutzstellung zum Sitzelement bleibendem Beinauflageelement erreicht wird, ist das Laschengetriebe so ausgebildet und ggf. auf das Kippgetriebe angepasst, dass das Beinauflageelement gegen Ende der ersten Bewegungsphase derart ausgerichtet ist, dass die Beinauflagefläche nach vorne hin abfällt.

Das Laschengetriebe weist vorzugsweise mindestens eine bezogen auf eine Möbellängsrichtung vordere und eine hintere Schwenklasche auf, die jeweils einerseits um voneinander abweichende erste und zweite Schwenkachsen an einem sitzelementseitigen Anbindungsabschnitt und jeweils andererseits um voneinander abweichende dritte und vierte Schwenkachsen an einem beinauflagenelementseitigem Anbindungsabschnitt schwenkbeweglich angebracht sind. Diese vier Schwenkachsen sind vorzugsweise derart uneinheitlich zueinander beabstandet, dass sie einen Bewegungspfad für das Beinauflageelement definieren und dabei gleichzeitig eine Schwenkbewegung bewirken, um in der Staustellung und der Nutzstellung jeweils ideale Ausrichtungen der Beinauflagefläche zur Sitzfläche zu gewährleisten.

Die Anbindungsabschnitte der Schwenklaschen sind vorzugsweise fester Teil der zur Sitzfläche bzw. der Beinauflagefläche ortsfesten Tragstrukturen des Sitzelements bzw. des Beinauflageelements. Allerdings kann alternativ grundsätzlich auch eine Relativbeweglichkeit eines Anbindungsabschnittes gegenüber der Tragstruktur seines jeweiligen Elements verbleiben, beispielsweise mittels eines weiteren Getriebes das Beinauflageelement um eine weitere Schwenkachse verschwenken zu können.

Der Elektromotor ist vorzugsweise als Linearaktuator mit einem translativ ausfahrbaren Läufer ausgebildet, wobei der Läufer vorzugsweise in einer Haupterstreckungsebene (+/- 10°) des Sitzelements ausgerichtet ist. Der Elektromotor kann insbesondere mit einem Ende um eine erste Motorachse schwenkbeweglich an einer Tragstruktur des Sitzelements angelenkt sein und mit einem zweiten Ende um eine zweite Motorachse schwenkbeweglich an einem Steuerglied angebracht sein, so dass die Linearbewegung zunächst in eine Schwenkbewegung umgeformt wird. Ein schwenkbewegliches Steuerglied eignet sich insbesondere als gemeinsames Steuerglied zur Anbindung des Kippgetriebes und des Laschengetriebes.

Zur Übertragung der Kraft vom Elektromotor auf das Laschengetriebe ist eine Schubstange vorgesehen, die ebenfalls vorzugsweise im Wesentlichen in einer Haupterstreckungsebene (+/- 10°) des Sitzelements ausgerichtet ist. Die Schubstange ist an einem Ende motorseitig angekoppelt, insbesondere durch die Anbindung an ein schwenkbewegliches Steuerglied, welches vom Motor verschwenkt wird. Anderenends kann die Schubstange unmittelbar an einer der Laschen des Laschengetriebes schwenkbar angelenkt sein, vorzugsweise auf die vordere der beiden Laschen, so dass die hintere Schwenklasche mittelbar aufgrund der vorderen Schwenklasche mitbewegt wird.

Sie kann jedoch stattdessen auch mittelbar über eine zusätzliche Schublasche auf die vordere Schwenklasche wirken. Diese zusätzliche Schublasche bietet die Möglichkeit, den Winkel, unter dem ein Schwenkmoment von der Schubstange auf die Schwenklasche übertragen wird, gezielt anzupassen, so dass die Kraftbeaufschlagung stets in vorteilhaftem Winkel erfolgt. Zusätzlich bietet die Schublasche auch eine hilfreiche Möglichkeit, um die gewünschte Entkoppelung der Relativstellung des Beinauflageelements zum Sitzelement zu realisieren, indem die fortgesetzte Bewegung der Schubstange durch die Schublasche gleichsam aufgenommen wird, aber nicht an das Beinauflageelement weitergegeben wird.

Zur Realisierung einersolchen Entkoppelung und/oderzur Krafteinkopplung in idealem Winkel ist die Schubstange vorzugsweise verschieblich an der Tragstruktur des Sitzelements geführt. Gleichzeitig ist die Schublasche um eine erste Schublaschenachse schwenkbeweglich an der Schubstange angebracht und um eine zweite Schublaschenachse schwenkbeweglich an der vorderen Schwenklasche angebracht. Die verschiebliche Führung ist insbesondere vorzugsweise mittels einer Kulissenführung mit einer Kulissenspur und einem entlang der Kulissenspur beweglichen Kulissengleiter, insbesondere in Form einer Kulissenrolle, realisiert. Sie weist einen Kulissenspurabschnitt auf, dessen Formgebung derart gestaltet ist, dass die erste Schublaschenachse gegenüber der Tragstruktur des Sitzelements und gegenüber der hierzu in dieser Phase ortsfesten zweiten Schublaschenachse im Wesentlichen eine Kreisbahn beschreibt. Die Kreisbahn führt dazu, dass die Schublasche um ihre zweite Schublaschenachse auf Seite der Schwenklasche schwenkt, diese dabei jedoch nicht mit einer Kraft beaufschlagt. Hierdurch wird die einheitliche Nutzstellung der Beinauflage in beiden Liegepositionen ermöglicht.

Um mit einem Laschengetriebe mit zwei Schwenklaschen, die beidseitig unmittelbar an den Tragstrukturen des Sitzelements und des Beinauflageelements angelenkt sind, den erforderlichen Weg des Beinauflageelements zurückzulegen, sollten die Länge der Schwenklaschen und der überstrichene Schwenkwinkel möglichst groß sein, bezogen auf die weniger weit schwenkende Schwenklasche vorzugsweise >110°, insbesondere vorzugsweise >120°. Dies bedeutet, dass die Schwenklaschen bei Anordnung des Beinauflageelements in seiner Nutzstellung sehr gestreckt sind und daher bei nicht ausreichend präzise gearbeiteter Herstellung des Beschlages die Gefahr des Überschlags droht.

Um dies zu verhindern, kann vorgesehen sein, dass das Laschengetriebe eine Sicherungsstrebe aufweist, die um eine fünfte Schwenkachse schwenkbeweglich an der hinteren Schwenklasche und um eine sechste Schwenkachse schwenkbeweglich an der vorderen Schwenklasche angebracht ist. Dabei sind diese Schwenkachsen von der Verbindungslinie der ersten und dritten bzw. der zweiten und vierten Schwenkachse versetzt, um auch gegen Ende der ersten Bewegungsphase und danach eine vorteilhafte Position aufzuweisen, so dass ein Überschlag durch zu große Kraftbeaufschlagung des Beinauflageelements durch Hebelwirkung verhindert wird.

Da die erste bis vierte Schwenkachse vorzugsweise kein perfektes Parallelogramm bilden, ändert sich der Abstand der fünften und sechsten Schwenkachse während der Bewegung des Laschengetriebes. Damit dies nicht zu einem Blockieren des Laschengetriebes führt, ist vorzugsweise vorgesehen, dass die Sicherungsstrebe an der hinteren Schwenklasche oder an der vorderen Schwenklasche schwenkbeweglich und begrenzt verschieblich angebracht ist, so dass sie lediglich einen Minimalabstand zwischen der fünften und der sechsten Schwenkachse erzwingt, jedoch eine demgegenüber größere Beabstandung der Schwenkachsen nicht verhindert. Dies kann insbesondere durch Anbindung der Sicherungsstrebe mittels eines Langlochs erzielt werden.

In Hinblick auf das Kippgetriebe wird es als vorteilhaft angesehen, wenn dieses eine zum Sitzelement und zur Basis ortsfeste Hauptachse aufweist, um die das Sitzelement gegenüber der Basis verkippbar ist. Hierdurch ist eine sehr einfache und kostengünstige Art der Kippbeweglichkeit geschaffen. Der Antrieb über den gemeinsamen oder auch einen separaten Elektromotor erfolgt vorzugsweise über ein dem Kippgetriebe zugeordnetes und um eine erste Steuergliedachse verschwenkbares Steuerglied, an welchem ein Kulissengleiter, vorzugsweise in Form einer Kulissenrolle, vorgesehen ist, die im Zuge des Verschwenkens des Steuergliedes entlang einer Kulissenspur beweglich ist.

Das Steuerglied, welches vorzugsweise um eine zum Sitzelement ortsfeste Steuergliedachse drehbar ist, verschwenkt im Zuge seines Verschwenkens aufgrund seiner Führung entlang der Kulissenspur das Sitzelement im geringerem Maße mit. Durch die Formgebung der Kulissenspur kann dabei genau definiert werden, in welcher Phase der Gesamtbewegung sich das Sitzelement verlagern soll.

Wenn die Kulissenspur über einen Kulissenspurabschnitt verfügt, der die Form eines Kreisabschnitts bezogen auf die erste Steuergliedachse aufweist, führt eine Schwenkbewegung des Steuergliedes phasenweise zu keinem Verschwenken des Sitzelements, so dass in dieser Phase dann eine isolierte Bewegung des Beinauflageelements gegenüber dem Sitzelement stattfinden kann.

Die Kulissenspur ist vorzugsweise an einem kippfest an der Basis angebrachten Kulissenelement vorgesehen.

Üblicherweise weist ein erfindungsgemäßes Sitzmöbelstück weiterhin ein Rücklehnenelement mit einer Rücklehnenfläche auf, welches vorzugweise um einen Rücklehnenachse schwenkbeweglich an der Basis oder am Sitzelement angebracht ist.

Zum Antrieb desselben ist vorzugsweise ein weiterer Elektromotor vorgesehen, mittels dessen das Rücklehnenelement um die Rücklehnenachse schwenkbar ist. Dieser Elektromotor wird von einer Steuereinrichtung des Sitzmöbelstücks angesteuert, wobei die Ansteuerung vorzugsweise mit der Ansteuerung des Elektromotors oder der Elektromotoren erfolgt, die das Sitzelement und das Beinauflageelement verlagert. Insbesondere vorzugsweise erfolgt das Verschwenken des Rücklehnenelements in eine flachere Stellung in der zweiten Bewegungsphase, in der das Sitzmöbelstück die Schlafkonfiguration einnimmt.

Wie eingangs bereits erläutert, eignet sich der Beschlag eines erfindungsgemäßen Sitzmöbelstücks zur Anbringung an Sitzmöbelstücken verschiedener Breite und insbesondere auch für Sessel ohne Seitenwangen/Armlehnen, da die technisch komplexen Beschlagsbauteile und insbesondere die Schwenklaschen zentrisch angeordnet sind und wenig Bauraum benötigen. Dennoch kann ein erfindungsgemäßer Sessel natürlich auch Armlehnen aufweisen. Diese können entweder gemeinsam mit dem Rückenlehnenelement um die Rücklehnenachse verschwenkbar sein oder gemeinsam mit dem Sitzelement verschwenkt werden.

Die genannte Basis kann als in sich vollständig starre Basis ausgebildet sein oder auch eine Drehachse definieren, um die ein verlagerbarer Abschnitt der Basis zur Anbringung des Sitzelements gegenüber deinem Aufstellabschnitt, beispielsweise einer bodenseitigen Platte verlagerbar ist.

Bei einer besonderen Gestaltung des Sitzmöbelstücks weist dieses zwischen dem Aufstellabschnitt oder einem demgegenüber drehbaren Zwischenabschnitt einerseits und dem verlagerbaren Abschnitt der Basis andererseits ein Aufstehhilfen-Getriebe auf, durch das der verlagerbare Abschnitt zwischen einer Nutzposition und einer Aufstehposition verlagerbar ist. Dass Getriebe ist dafür vorgesehen, den eigentlichen Sitz, also das Sitzelement, das Beinauflageelement und das Rückenlehnenelement gemeinsam anzuheben und so ein erleichtertes Aufstehen zu gestatten.

Das Getriebe weist vorzugsweise zwei Getriebelaschen auf, die sich zwischen dem Aufstellabschnitt oder einem demgegenüber drehbaren Zwischenabschnitt und dem verlagerbaren Abschnitt erstrecken und die beidseitig um unterschiedliche Schwenkachsen verschwenkbar sind. Die Getriebelaschen verfügen vorzugsweise über eine in etwa gleiche Längen, so dass der Sitz in etwa parallel angehoben wird. Durch leicht unterschiedliche Längen oder durch Anordnung der Schwenkachsen in von einer Parallelogramm abweichender Form lässt sich erzielen, dass das Anheben mit einem leichten Verkippen nach vorne verbunden ist.

Die Getriebelaschen erstrecken sich vorzugsweise vom verlagerbaren Abschnitt nach hinten. Insbesondere vorzugweise erstrecken sich die beiden Getriebelaschen ausgehend vom Aufstellabschnitt oder dem Zwischenabschnitt in der Nutzposition des verlagerbaren Abschnitts schräg nach oben und in der Aufstehposition schräg nach unten.

Das Sitzelement mit der Beinauflage beschreibt beim Hochfahren der Aufstehhilfe aufgrund der Getriebelaschen in etwa einen Kreisbogen, wobei das Sitzelement hierbei bezogen auf die Möbellängsrichtung zunächst nach vorne und dann wieder nach hinten verlagert wird. Dies hat sich als sehr vorteilhaft herausgestellt, um trotz des unter dem Sitzelement befindlichen Beinauflageelements ein kollisionsfreies Hochfahren zu gestatten.

Vorzugsweise ist an einer der Getriebelaschen ein Ruderhorn, also ein starr angebrachter Momenteneinkopplungspunkt abseits der Verbindung der Schwenkachsen, vorgesehen, an dem ein Elektromotor zum Anheben des Sitzes angreift. Diese Elektromotor ist vorzugweise unterhalb des Sitzelements angeordnet, vorzugsweise auf der gegenüberliegenden Seite zum exzentrisch angeordneten Elektromotors zur Verlagerung des Beinauflageelements. Zwar wird dieser Ort bei Varianten ohne Aufstehhilfe bevorzugt für einen Rückenlehnen-Elektromotor verwendet. Dieser kann jedoch auch in der Rückenlehne selbst angeordnet sein.

Bei einer Gestaltung des Sitzmöbelstücks, bei der das Sitzelement um eine vertikale Achse gegenüber dem Aufstellabschnitt der Basis verdrehbar ist, ist vorzugsweise eine Wirkkoppelung mit der Aufstehhilfe vorgesehen, die dafür sorgt, dass die Drehbeweglichkeit blockiert wird, wenn das Aufstehhilfe-Getriebe den Sitz aus der Nutzposition herausbewegt.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1A bis 1C zeigen exemplarisch ein erfindungsgemäßes Sitzmöbelstück in drei Konfigurationen.
Fig. 2A und 2B zeigen das Sitzmöbelstück und seinen Möbelbeschlag in einer Sitzkonfiguration des Sitzmöbelstücks.
Fig. 3A und 3B zeigen das Sitzmöbelstück und seinen Möbelbeschlag in einem Zwischenzustand während der Überführung in einer ersten Liegekonfiguration, nämlich der Fernsehkonfiguration.
Fig. 4A und 4B zeigen das Sitzmöbelstück und seinen Möbelbeschlag in der Fernsehkonfiguration.
Fig. 5A und 5B zeigen das Sitzmöbelstück und seinen Möbelbeschlag in einer zweiten Liegekonfiguration, der Schlafkonfiguration.
Fig. 6A und 6B zeigen eine Variante des Sitzmöbelstücks mit einer Aufstehhilfe-Funktion.

### DETAILLIERTE BESCHREIBUNG DER AUSFÜHRUNGSBEISPIELE

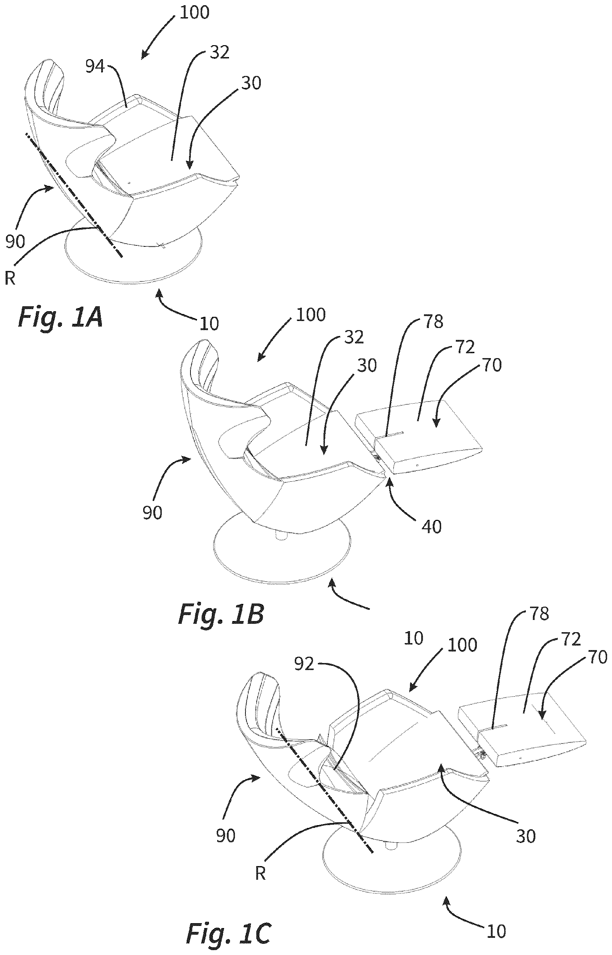

Die Fig. 1A und 1C zeigen beispielhaft ein erfindungsgemäßes Sitzmöbelstück, welches zwischen drei möglichen Konfigurationen verfahren werden kann, in der seine Elemente jeweils in verschiedener Position und Ausrichtung zu einer Basis 10 angeordnet sind. Fig. 1A zeigt eine Sitzkonfiguration. Fig. 1B zeigt eine erste Liegekonfiguration mit ausgefahrenem Beinauflageelement 70, die so genannte Fernseh-Konfiguration. Fig. 1C zeigt eine zweite Liegekonfiguration nach fortgesetztem Verschwenken des Sitzelements 30 und flacher ausgerichtetem Rückenlehnenelement 90 mit einer Rücklehnenfläche 92. Dies ist die so genannte Schlafkonfiguration.

In der Stellung der Fig. 1A, der Sitzkonfiguration, ist ein aufrechtes Sitzen auf der Sitzfläche 32 des Sitzelements 30 möglich. Von hier aus kann das Sitzmöbelstück 100 in die erste Liegekonfiguration verfahren werden, indem das Beinauflageelement 70 aus seiner Staustellung der Fig. 1A in seine Nutzstellung vor dem Sitzelement 30 und der Sitzfläche 32 verlagert wird. Hierbei findet ein Laschengetriebe 40 Verwendung, welches im Weiteren noch detailliert erläutert wird und welches über Schwenklaschen 46, 48 verfügt, die in der Staustellung der Fig. 1 in dem in Fig. 1B erkennbaren Schlitz 78 des Beinauflageelements 70 angeordnet waren. Dieses ist hierfür auch im Bereich des Beschlages mit einem zentrischen Schlitz 79 versehen.

Ausgehend von der ersten Liegekonfiguration der Fig. 1B kann das Sitzmöbelstück 100 in die zweite Liegekonfiguration der Fig. 1C verfahren werden. Das Sitzelement 30 und die Sitzfläche 32 bleiben hierbei relativ zum Beinauflageelement 70 und der Beinauflagefläche 72 ortsfest. Gemeinsam miteinander verschwenken jedoch das Sitzelement 30 und das Beinauflageelement 70 weiter, so dass die Sitzfläche in vertikaler Hinsicht unterhalb der Beinauflagefläche 72 angeordnet ist. In dieser Phase wird mittels eines getrennten Elektromotors auch das Rückenlehnenelement 90 in eine flachere Ausrichtung verfahren.

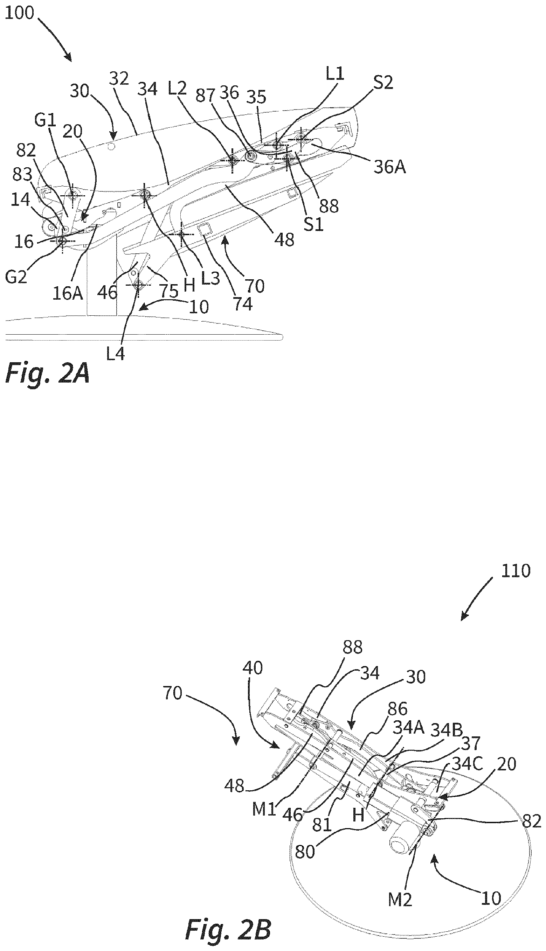

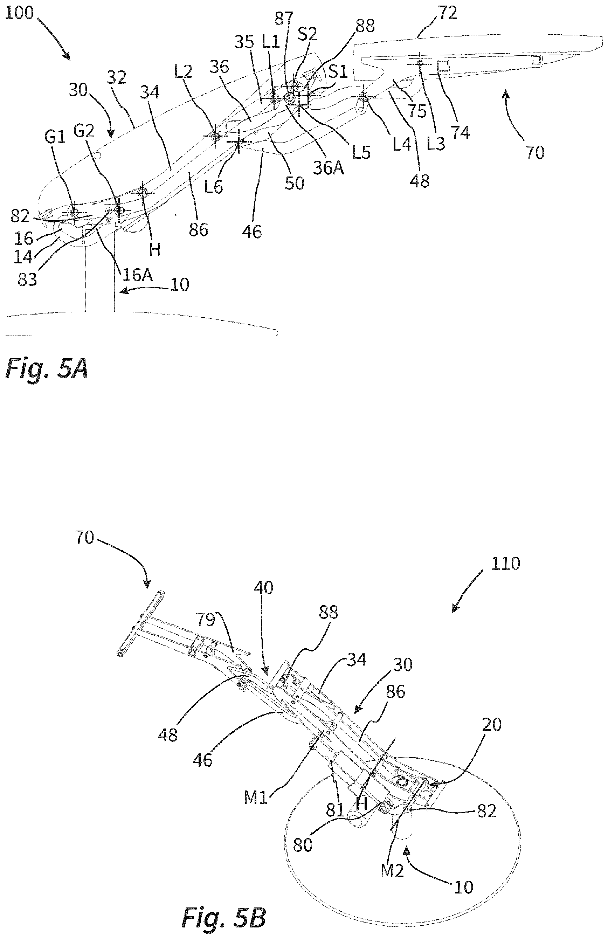

Die Fig. 2A und 2B bis 5A und 5B verdeutlichen anhand jeweils einer geschnittenen Seitendarstellung und einer ungeschnittenen Darstellung des Beschlages 110 des Sitzmöbelstücks 100 die genannten Wechsel der Konfigurationen. Dabei sind in diesen Darstellungen das Rückenlehnenelement 90 und die korrespondierenden Beschlagsteile nicht gezeigt.

Die Fig. 2A und 2B entsprechen der Konfiguration der Fig. 1A, also der Sitzkonfiguration. In dieser Konfiguration ist das Beinauflageelement 70 unterhalb des Sitzelements 30 und vor allen Dingen unterhalb von dessen Sitzfläche 32 angeordnet. Dabei ist das Beinauflageelement 70 nach vorne ansteigend ausgerichtet, um auf diese Art und Weise sich eng an die Unterseite des Sitzelements 30 anlegen zu können.

Die Anbindung des Beinauflageelements 70 an das Sitzelement 30 erfolgt über jeweilige Tragstrukturen 34, 74. Diese verfügen jeweils überAnbindungsabschnitte 35, 75, an denen um Schwenkachsen L1, L2 auf Seiten des Sitzelements 30 eine hintere und eine vordere Schwenklasche 46, 48 des Laschengetriebes 40 schwenkbar angelenkt sind. Die Schwenklaschen 46, 48 sind an ihrem jeweils gegenüberliegenden Ende schwenkbar um die Schwenkachsen L3, L4 am Anbindungsabschnitt 75 der Tragstruktur 74 des Beinauflageelements 70 angelenkt. Die Schenkachsen L1, L2, L3, L4 und ihre jeweiligen Abstände definieren den Bewegungspfad des Beinauflageelements 70 relativ zum Sitzelement, wobei durch Unterschiede in den Abständen L1-L3, L2-L4, L1-L2, L3-L4 bewirkt wird, dass das Beinauflageelement 70 bei der Verlagerung entlang dieses Pfades zwangsläufig auch verschwenkt wird.

Am hinteren Ende des Sitzelements 30 ist dieses an einer zur ortsfesten Aufstellung vorgesehenen Basis 10 schwenkbar angelenkt, und zwar um die Hauptachse H, definiert durch einen Zapfen 37. Hier kann alternativ auch ein Laschengetriebe ähnlich dem Laschengetriebe 40 vorgesehen sein.

Zur Überführung des Sitzmöbelstücks 100 in die in den Fig. 1B und 1C dargestellten Konfigurationen sind eine Reihe von Motor- und Getriebeelementen vorgesehen.

Das Sitzmöbelstück 100 verfügt über einen gemeinsamen Elektromotor 80 in Form eines Linearaktuators zur Verlagerung des Sitzelements 30 gegenüber der Basis 10 einerseits und zur Verlagerung des Beinauflageelements 70 gegenüber dem Sitzelement 30 andererseits. Der Elektromotor 80 verfügt über einen ausfahrbaren Läufer 81, der endseitig um eine erste Motorenschwenkachse M1 schwenkbar an der Tragstruktur 34 des Sitzelements 30 angebracht ist. Das gegenüberliegende Ende des Elektromotors 80 wird durch die zweite Motorschwenkachse M2 definiert, an der der Elektromotors 80 schwenkbeweglich mit einem Steuerglied 82 verbunden ist. Dieses Steuerglied 82 ist drehbar an zwei Wangen 34A, 34B der Tragstruktur 34 angebracht, die einen Zwischenraum 34C definieren, in welchem insbesondere die Schwenklaschen 46, 48 angeordnet sind.

Das Steuerglied 82 verfügt über zwei Ausleger beidseitig der Wangen 34A, 34B. Der Ausleger auf Seiten des Elektromotors 80 ist zur Anbindung des Motors vorgesehen. Der gegenüberliegende Ausleger, der in Fig. 2A zu sehen ist, dient zur Anbindung sowohl eines Kippgetriebes 20 zum Verkippen des Sitzelements 30, als auch des bereits genannten Laschengetriebes 40, dessen Hauptbestandteile die vordere und die hintere Schwenklasche 48, 46 sind. Der Ausleger des Steuergliedes 82 auf der dem Elektromotor 80 gegenüberliegenden Seite verfügt über einen Kulissengleiter 83 in Form einer Kulissenrolle, die in einer zur Basis gehörigen Kulissenspur 16 eines Kulissenelements 14 angeordnet ist. Das Steuerglied 82 ist als Ganzes, also gemeinsam mit den genannten Auslegern, um die Steuergliedachse G1 am Sitzelement 30 verschwenkbar. Am gegenüberliegenden distalen Ende des Steuergliedes 82 motorabgewandten Auslegers ist eine Schubstange 86 angebracht, die der Kraftübertragung vom Steuerglied 82 an das Laschengetriebe 40 dient. Zu diesem Zweck ist die Schubstange 86 auf der der Steuergliedachse G2 gegenüberliegenden vorderen Seite mittels eines Kulissengleiters 87 in einer basisseitigen Kulissenspur 36 geführt.

Am distalen Ende der Schubstange 86 verfügt diese über eine hieran angebundene Schublasche 88, die um die Schublaschenachse S1 schwenkbar angelenkt ist und die mit ihrem gegenüberliegenden Ende schwenkbar um die Schublaschenachse S2 an der vorderen Schwenklasche 48 angelenkt ist. Hierdurch kann die Kraft des Elektromotors 80 verwendet werden, um das Beinauflageelement 70 mittels der vorderen Schwenklasche 48 bezogen auf Fig. 2A gegen den Uhrzeigersinn auszuschwenken. Dadurch, dass die Schubstange 86 nicht unmittelbar an der vorderen Schwenklasche 48 angelenkt ist, kann eine Kraftbeaufschlagung in einem vorteilhaften Winkel erfolgen, was insbesondere aufgrund der großen Gesamtschwenkwinkel der Schwenklaschen 46, 48 von Relevanz ist. Weiterhin wird hierdurch auch die Grundlage dafür geschaffen, dass bei der Überführung aus der ersten Liegekonfiguration in die zweite Liegekonfiguration keine Relativbewegung zwischen dem Beinauflageelement und dem Sitzelement mehr erfolgt oder diese zumindest nur in sehr geringem Maße gegeben ist.

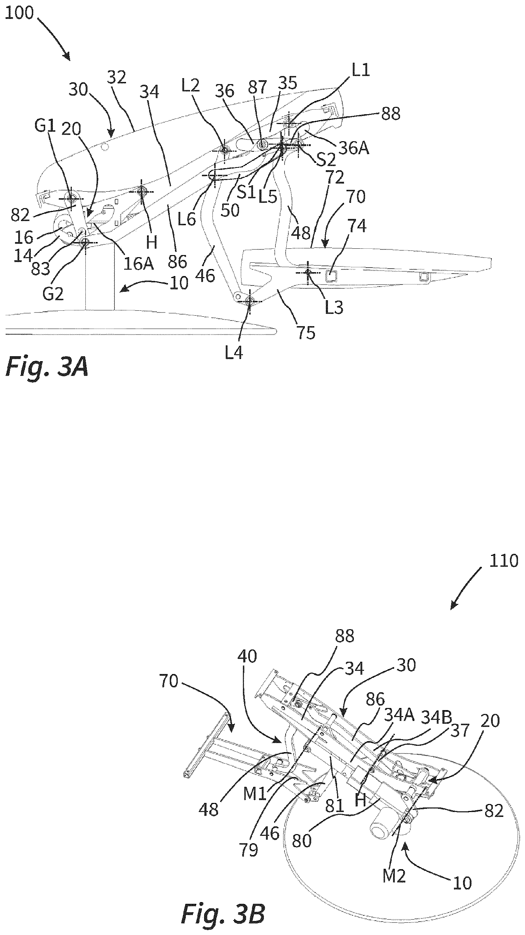

Wenn ausgehend von der Konfiguration der Fig. 1A sowie 2A und 2B der Elektromotor aktiviert wird, so stellt sich auf dem Weg in die erste Liegekonfiguration eine Zwischensituation entsprechend der Fig. 3A und 3B ein. Es ist zu erkennen, dass die Schwenkbewegung des Steuergliedes 82, die durch ein Ausfahren des Läufers 81 bewirkt wurde, in zweierlei Hinsicht die Stellung der Elemente des Sitzmöbelstücks 100 beeinflusst hat.

Zum einen ist die Schubstange 86 nach vorne in Richtung der vorderen Sitzkante verlagert worden, was unter Vermittlung der Schublasche 88 zu einem Ausschwenken der Schwenklasche 48 und mittelbar auch der Schwenklasche 46 geführt hat. Durch die unterschiedliche Beabstandung der Schwenkachsen L1, L2 bzw. L3, L4 und/oder durch die unterschiedlichen Längen der Schwenklaschen 46, 48 ist das Beinauflageelement im Zwischenzustand der Fig. 3A nicht mehr nach vorne ansteigend, sondern quasi horizontal ausgerichtet.

Damit das Beinauflageelement 70 überhaupt ohne zwischenzeitlichen Bodenkontakt sicher ausgefahren werden kann, hat jedoch auch ein leichtes Verschwenken des Sitzelements 30 bezogen auf Fig. 3A gegen den Uhrzeigersinn stattgefunden, indem der Kulissengleiter 83 entlang der Kulissenspur 16 verlagert wurde und dabei das den Schwenkwinkel des Sitzelements 30 definierende Dreieck aus den Achsen G1, H und dem Kulissengleiter 83 verändert hat. Der vordere Teil des Sitzelements 30 ist hierdurch leicht angehoben worden, so dass selbst in diesem Zustand mit maximal abgesenktem Beinauflageelement 70 keine Bodenkollision stattfindet.

Im Zustand der Fig. 3A, 3B ist zu erkennen, dass die Schwenklaschen 46, 48 durch eine weitere verbindende Strebe, die gestrichelt eingezeichnete Sicherungsstrebe 50, miteinander verbunden sind. Diese Strebe ist um Schwenkachsen L5, L6 schwenkbar an beiden Schwenklaschen 46, 48 angebracht, hat jedoch gegenüber einer der beiden etwas Spiel, was beispielsweise über ein Langloch erreicht werden kann. Die Sicherungslasche soll beim fortgesetzten Ausfahren des Beinauflageelements und der damit einhergehenden starken Streckung der Schwenklaschen 46, 48 gewährleisten, dass das Beinauflageelement 70 nicht überschlägt, wie im Weiteren noch erläutert ist.

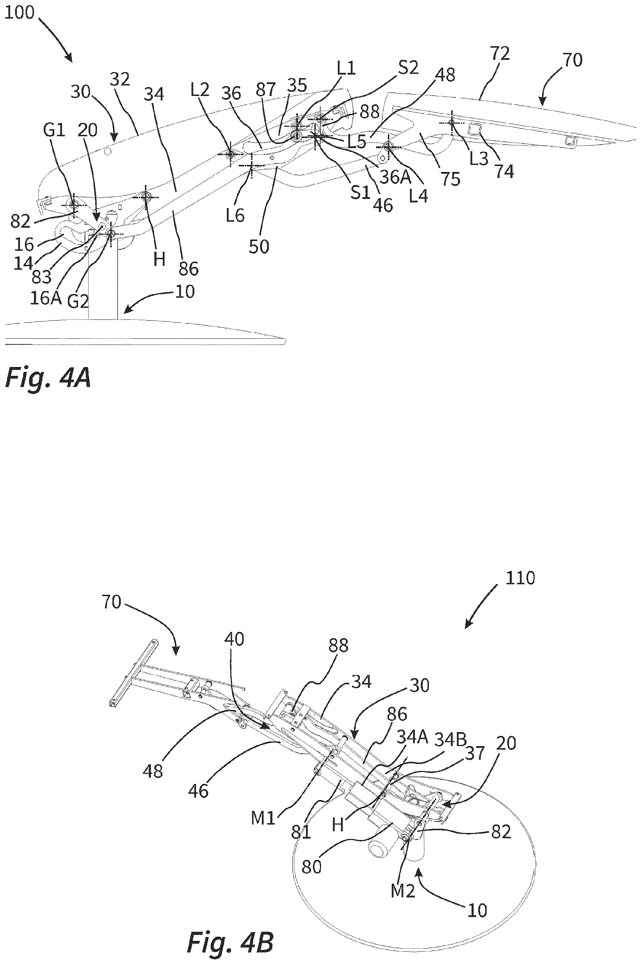

Bei der fortgesetzten Bewegung wird die erste Liegekonfiguration erreicht, wie in den Fig. 4A und 4B sowie in Fig. 2B dargestellt ist. Zwischen den Stellungen der Fig. 3A, 3B und 4A, 4B, 2B ist das Sitzelement 30 ortsfest verblieben. Dies wurde erreicht durch einen mittleren Kulissenspurabschnitt 16A der Kulissenspur 16, der eine Kreisabschnittsform zur Steuergliedachse G1 darstellt. Dieser Kulissenspurabschnitt 16A ermöglicht es, dass in dieser Phase zwischen den Fig. 3A, 3B und 4A, 4B das Steuerglied 82 weiterschwenkt, dies das Sitzelement 30 jedoch nicht beeinflusst.

Das Beinauflageelement 70 ist hingegen bei der Überführung in den Zustand der Fig. 4A, 4B, 2B in seine relative Endlage zum Sitzelement 30 gelangt. In diesem Zustand fluchten die Sitzfläche 32 und die Beinauflagefläche 72 im Wesentlichen miteinander. Es stellt sich also keine störende Stufe zwischen ihnen ein. Die Beinauflagefläche 72 ist in dieser Konfiguration nach vorne leicht abfallend ausgerichtet, was in der typischen Fernsehsituation als angenehm empfunden wird.

In der Relativstellung des Beinauflageelements 70 gegenüber dem Sitzelement 30, wie er sich ab Fig. 4A, 4B einstellt, ist das Viereck, dass durch die Schwenkachsen L1, L2, L3, L4 gebildet wird, sehr flach, so dass die Schwenkachsen fast miteinander fluchten. Hierdurch besteht die Gefahr, dass bei einer hohen Kraftbeaufschlagung des Beinauflageelements die Schwenkachse L4 nach oben klappt, so dass die Schwenklaschen 46, 48 einander kreuzen. Dies wird jedoch selbst bei ungenauer Fertigung des Beschlages durch die Sicherungsstrebe 50 unterbunden, die aufgrund der Anordnung ihrer Schwenkachsen L5, L6 versetzt zu den jeweiligen Verbindungslinien L1-L3, L2-L4 ein solches Umklappen verhindert.

Bei fortgesetztem Betrieb des Elektromotors 80 stellt sich der Zustand der Fig. 5A, 5B und 1C ein, welches die zweite Liegekonfiguration ist. Beim Übergang von der ersten Liegekonfiguration der Fig. 4A, 4B zur zweiten Liegekonfiguration der Fig. 5A, 5B ist es nicht zu einer Relativverlagerung des Beinauflageelements 70 zum Sitzelement 30 gekommen. Hierfür ist verantwortlich, dass in einem letzten Kulissenspurabschnitt 36A der Kulissenspur 36 diese eine Formgebung aufweist, die dazu führt, dass die Schublaschenachse S1 eine Kreisbogenbewegung um die Schublaschenachse S2 durchläuft. Eine Kraftbeaufschlagung über die Schublasche 88 auf die Schublaschenachse S2 findet jedoch nicht mehr statt und somit auch keine Verlagerung des Beinauflageelements 70 gegenüber dem Sitzelement 30.

Am Kippgetriebe 20 jedoch hat in dieser letzten Phase der Kulissengleiter 83 den entkoppelnden Kulissenspurabschnitt 16A verlassen, so dass in dieser letzten Phase die Schwenkbewegung des Steuergliedes 82 ein Verkippen des Sitzelements und damit auch des Beinauflageelements 70 bewirkt, die aufgrund der starken Krümmung in diesem Endabschnitt der Kulissenspur 16 sehr stark ausfällt. Das Sitzelement 30 wird in dieser letzten Phase nochmals etwa um 10° bis 15° verschwenkt.

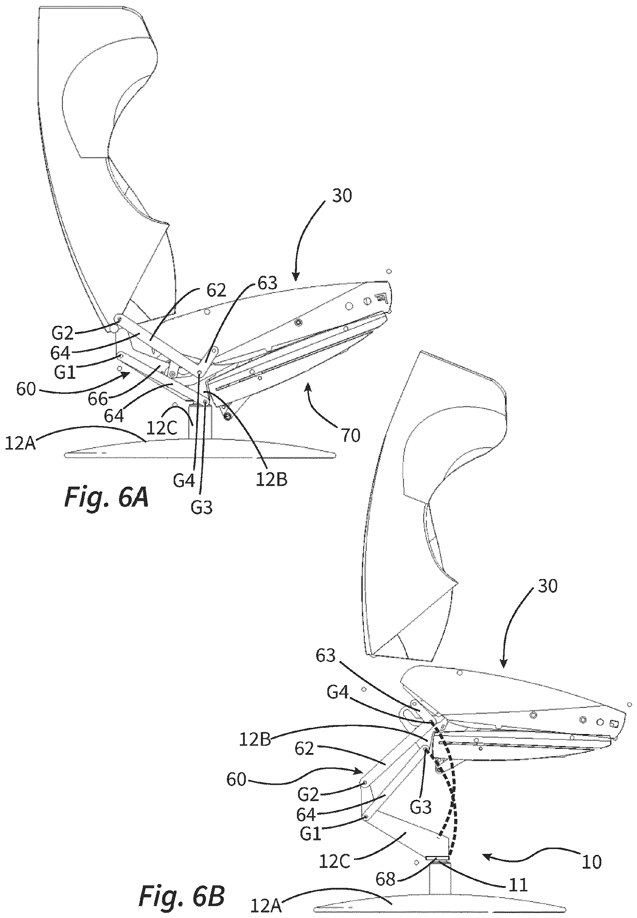

Die erfindungsgemäße Gestaltung stellt ein elektrifiziertes Sitzmöbelstück zur Verfügung, welches aufgrund der zentrischen Anordnung der meisten mechanischen Elemente und insbesondere der Schwenklaschen im Betrieb nur mit einer sehr geringen Verletzungsgefahr einhergeht und an bestehenden Modellen von Sitzmöbelstücken nur geringe Änderungen erforderlich macht, um diese in elektrifizierter Ausgestaltung mit Beinauflageelement anbieten zu können. Insbesondere stellt die Erfindung auch Maßnahmen zur Verfügung, um den erforderlichen Bauraum eines erfindungsgemäßen Beschlages dadurch gering zu halten, dass nur ein Motor für beide Bewegungen verantwortlich ist, wobei durch zeitweise Entkopplung trotzdem eine zweckmäßige Bewegungsreihenfolge bewirkt werden kann. Fig. 6A und 6B zeigen eine Variante des Sitzmöbelstücks mit einer Aufstehhilfe-Funktion. Das Sitzmöbelstück entspricht in technischer Hinsicht weitgehend dem erläuterten. Relevante Unterschiede ergeben sich nur hinsichtlich der Basis 10.

Bei dieser Gestaltung weist die Basis 10 einen bodenseitigen Aufstellabschnitt 12A auf, an dem um eine vertikale Drehachse drehbar ein Zwischenabschnitt 12C mit gekröpfter Formgebung angebracht ist. Zwischen diesem Zwischenabschnitt 12C und einem verlagerbaren Abschnitt 12B, der mit dem Kulissenelement 14 identisch ist, ist das Aufstehhilfen-Getriebe 60 vorgesehen. Dieses umfasst zwei Getriebelaschen 62, 64, die am Zwischenabschnitt 12C und am verlagerbaren Abschnitt 12B um Schwenkachsen G1, G2, G3, G4 verschwenkbar sind. Die Getriebelasche 62 ist an einem Ende mit einem Ruderhorn 63 ausgebildet, an dem in nicht dargestellter Weise ein zweiter Elektromotor angreift, der in etwa spiegelbildlich zum beschriebenen Elektromotor 80 auf der gegenüberliegenden Seite bezogen auf die Möbelquerrichtung angeordnet ist.

Fig. 6A zeigt den Nutzzustand, in welchem Das Sitzmöbelstück in der beschriebenen Art verwendet werden kann. Die Getriebelaschen 62, 64 erstrecken sich vom Zwischenabschnitt 12C nach vorne und unten. Das Sitzelement ruht auf einer Druckfläche 68 am Zwischenabschnitt 12C, durch deren Kraftbeaufschlagung eine Drehbremse zwischen dem Zwischenabschnitt 12C und dem bodenseitigen Aufstellabschnitt 12A freigegeben ist, so dass der Sitz drehbar ist.

Wird nun der Elektromotor der Aufstehhilfen-Funktion aktiviert, so koppelt dieser ein Moment in die Getriebelasche 62 ein, so dass der verlagerbare Abschnitt 12B angehoben wird und dabei durch die Anordnung der Schwenkachsen G1, G2, G3, G4 auch leicht nach vorne gekippt wird. Die Bewegung der Schwenkachsen G3, G4 und des verlagerbaren Abschnitts 12B erfolgt dabei entlang der in Fig. 6B gestrichelt dargestellten Pfade. Es ist ersichtlich, dass das Sitzelement 30 mit dem Beinauflageelement 70 entlang einer Kreisbahn verlagert wird.

Mit Beginn des Anhebens des Sitzelements 30 wird dieses auch von der Druckfläche 68 angehoben, die bis dahin die Freigabe der Drehung des Zwischenabschnitts 12C gegenüber dem bodenseitigen Aufstellabschnitt 12A ermöglicht hat. Es stellt sich also eine Drehsperrung ein, durch die das Aufstehen erleichtert ist.

## Patentansprüche

1. Sitzmöbelstück (100) mit den folgenden Merkmalen:
a. das Sitzmöbelstück (100) verfügt über eine Basis (10) zur ortsfesten Aufstellung, und
b. das Sitzmöbelstück (100) verfügt über ein gegenüber der Basis (10) kippbewegliches Sitzelement (30) mit einer Sitzfläche (32) und einer Tragstruktur (34), und
c. das Sitzmöbelstück (100) verfügt über ein gegenüber dem Sitzelement (30) bewegliches Beinauflageelement (70) mit einer Beinauflagefläche (72) und einer Tragstruktur (74), und
d. das Beinauflageelement (70) ist mittels eines Laschengetriebes (40) mit dem Sitzelement (30) verbunden, wobei mittels des Laschengetriebes (40) das Beinauflageelement (70) zwischen einer Staustellung unter dem Sitzelement (30) und einer Nutzstellung vor dem Sitzelement (30) beweglich ist,
**gekennzeichnet durch** die folgenden zusätzlichen Merkmale:
e. das Sitzmöbelstück verfügt über einen Elektromotor (80) unterhalb der Sitzfläche (32), welcher über eine Schubstange (86) auf das Laschengetriebe (40) wirkt, und
f. zur Anbindung des Beinauflageelements (70) an das Sitzelement (30) weist das Laschengetriebe (40) mindestens eine Schwenklasche (48) auf, die bei der Überführung des Beinauflageelements (70) aus der Staustellung in die Nutzstellung phasenweise in einem die Beinauflagefläche (72) partiell durchbrechenden Schlitz (78) angeordnet ist.

2. Sitzmöbelstück (100) nach Anspruch 1 mit den folgenden zusätzlichen Merkmalen:
a. zur Steuerung einer Kippbewegung des Sitzelements (30) ist ein Kippgetriebe (20) vorgesehen, welches mittels des Elektromotors (80) angetrieben werden kann, und
b. der Elektromotor (80) wirkt mittels eines gemeinsamen Steuergliedes (82) auf das Kippgetriebe (20) und das Laschengetriebe (40),
vorzugsweise mit den zusätzlichen Merkmalen:
c. das Kippgetriebe (20) ist dafür ausgebildet, bei der Bewegung des Steuergliedes (82) aus einer Anfangsstellung in eine Endstellung eine Verlagerung des Sitzelements (30) gegenüber der Basis (10) zu bewirken, wobei weiterhin das Kippgetriebe (20) derart ausgebildet ist, dass in mindestens einer Bewegungsphase des Steuergliedes (82) die Relativstellung des Sitzelements (30) zur der Basis (10) unverändert bleibt, und/oder
d. das Laschengetriebe (40) ist dafür ausgebildet, bei der Bewegung des Steuergliedes (82) aus einer Anfangsstellung in eine Endstellung eine Verlagerung des Beinauflageelements (70) gegenüber dem Sitzelement (30) zu bewirken, wobei das Laschengetriebe (40) weiterhin derart ausgebildet ist, dass in mindestens einer Bewegungsphase des Steuergliedes (82) die Relativstellung des Beinauflageelements (70) zum Sitzelement (30) unverändert bleibt.

3. Sitzmöbelstück (100) nach einem der vorstehenden Ansprüche mit dem folgenden zusätzlichen Merkmal:
a. die Bewegung des Sitzelements (30) gegenüber der Basis (10) ist mit der Bewegung des Beinauflageelements (70) gegenüber dem Sitzelement (30) derart gekoppelt, dass
i. in einer ersten Bewegungsphase das Beinauflageelement (70) aus der Staustellung in die Nutzstellung überführt wird, und
ii. in einer sich daran anschließenden zweiten Bewegungsphase das Sitzelement (30) gemeinsam mit dem Beinauflageelement (70) gegenüber der Basis (10) verkippt wird, während das Beinauflageelement (70) ortsfest zum Sitzelement (30) verbleibt.

4. Sitzmöbelstück (100) nach Anspruch 3 mit dem folgenden zusätzlichen Merkmal:
a. in einer Stellung gegen Ende der ersten Bewegungsphase und vor Beginn der zweiten Bewegungsphase ist das Beinauflageelement (70) derart ausgerichtet, dass die Beinauflagefläche (72) nach vorne hin abfällt,
vorzugsweise mit dem zusätzlichen Merkmal:
b. in einer Stellung gegen Ende der zweiten Bewegungsphase ist das Beinauflageelement (70) derart ausgerichtet, dass die Beinauflagefläche (72) horizontal ausgerichtet ist oder nach hinten hin abfällt.

5. Sitzmöbelstück (100) nach einem der vorstehenden Ansprüche mit dem folgenden zusätzlichen Merkmal:
a. das Laschengetriebe (40) weist mindestens eine vordere und eine hintere Schwenklasche (48, 46) auf, die jeweils einerseits um voneinander abweichende erste und zweite Schwenkachsen (L1, L2) an einem sitzelementseitigen Anbindungsabschnitt (35) und jeweils andererseits um voneinander abweichende dritte und vierte Schwenkachsen (L3, L4) an einem beinauflagenelementseitigen Anbindungsabschnitt (75) schwenkbeweglich angebracht sind,
vorzugsweise mit den zusätzlichen Merkmalen:
b. der sitzelementseitige Anbindungsabschnitt (35) wird durch einen Teil der Tragstruktur (34) des Sitzelements (30) gebildet, die ortsfest zur Sitzfläche (32) vorgesehen ist, und/oder
c. der beinauflagenelementseitige Anbindungsabschnitt (75) wird durch einen Teil der Tragstruktur (74) des Beinauflageelements (70) gebildet, die ortsfest zur Beinauflagefläche (72) vorgesehen ist.

6. Sitzmöbelstück (100) nach Anspruch 5 mit dem folgenden zusätzlichen Merkmal:
a. die Schubstange (86), über die der Elektromotor auf das Laschengetriebe (40) wirkt, ist derart am Laschengetriebe (40) angebunden, dass sie unmittelbar oder über eine zusätzliche Schublasche (88) auf die vordere Schwenklasche (48) wirkt, wobei die hintere Schwenklasche (46) mittelbar aufgrund der Bewegung der vorderen Schwenklasche (48) mitbewegt wird,
vorzugsweise mit den zusätzlichen Merkmalen:
b. die Schubstange (86) ist verschieblich an der Tragstruktur (34) des Sitzelements (30) geführt, und
c. an der Schubstange (86) ist die Schublasche (88) um eine erste Schublaschenachse (S1) schwenkbeweglich angebracht und an der vorderen Schwenklasche (48) ist die Schublasche (88) um eine zweite Schublaschenachse (S2) schwenkbeweglich angebracht.

7. Sitzmöbelstück (100) nach einem der vorstehenden Ansprüche mit dem folgenden zusätzlichen Merkmal:
a. die Schubstange (86) ist mittels einer Kulissenführung mit einer Kulissenspur (36) und einem entlang der Kulissenspur (36) beweglichen Kulissengleiter (87) geführt,
vorzugsweise mit dem zusätzlichen Merkmal:
b. die Kulissenspur (36) weist einen Kulissenspurabschnitt (36A) auf, dessen Formgebung derart gestaltet ist, dass die erste Schublaschenachse (S1) gegenüber der Tragstruktur (34) des Sitzelements (30) und gegenüber der zweiten Schublaschenachse (S2) eine Kreisbahn beschreibt.

8. Sitzmöbelstück (100) nach einem der Ansprüche 5 bis 7 mit den folgenden zusätzlichen Merkmalen:
a. das Laschengetriebe (40) weist eine Sicherungsstrebe (50) auf, die um eine fünfte Schwenkachse (L6) schwenkbeweglich an der hinteren Schwenklasche (46) und um eine sechste Schwenkachse (L5) schwenkbeweglich an der vorderen Schwenklasche (48) angebracht ist, und
b. eine Verbindungslinie zwischen der ersten und der dritten Schwenkachse (L1, L3) schließt mit einer Verbindungslinie zwischen der ersten und der fünften Schwenkachse (L1, L5) einen Winkel von mindestens 15° ein, und
c. eine Verbindungslinie zwischen der zweiten und der vierten Schwenkachse (L2, L4) schließt mit einer Verbindungslinie zwischen der zweiten und der sechsten Schwenkachse (L2, L6) einen Winkel von mindestens 15° ein.
vorzugsweise mit dem zusätzlichen Merkmal:
d. die Sicherungsstrebe (50) ist an der hinteren Schwenklasche (46) oder an der vorderen Schwenklasche (48) schwenkbeweglich und begrenzt verschieblich angebracht, so dass sie einen Minimalabstand zwischen der fünften und der sechsten Schwenkachse (L5, L6) erzwingt.

9. Sitzmöbelstück (100) nach einem der vorstehenden Ansprüche mit den folgenden zusätzlichen Merkmalen:
a. das Kippgetriebe (20) weist eine zum Sitzelement (30) und zur Basis (10) ortsfeste Hauptachse (H) auf, um die das Sitzelement (30) gegenüber der Basis (10) verkippbar ist, und
b. dem Kippgetriebe (20) ist ein durch den Elektromotor (80) um eine erste Steuergliedachse (G1) verschwenkbares Steuerglied (82) zugeordnet, an welchem ein Kulissengleiter (83) vorgesehen ist, der im Zuge des Verschwenkens des Steuergliedes (82) entlang einer Kulissenspur (16) beweglich ist, so dass das Sitzelement (30) hierdurch um die Hauptachse (H) verkipptwird.
vorzugsweise mit dem zusätzlichen Merkmal:
a. die Kulissenspur (16) weist einen Kulissenspurabschnitt (16A) auf, der die Form eines Kreisabschnitts bezogen auf die erste Steuergliedachse (G1) aufweist.

10. Sitzmöbelstück (100) nach Anspruch 9 mit den folgenden zusätzlichen Merkmalen:
a. die Kulissenspur (16) ist an einem kippfest an der Basis (10) vorgesehenen Kulissenelement (14) vorgesehen, und
b. die Steuergliedachse (G1) ist ortsfest zum Sitzelement (30) vorgesehen.

11. Sitzmöbelstück (100) nach einem der vorstehenden Ansprüche mit dem folgenden zusätzlichen Merkmal:
a. der Elektromotor (80) ist als Linearmotor mit einem ausfahrbaren Läufer (81) ausgebildet, wobei der Läufer (81) in einer Haupterstreckungsebene (+/- 10°) des Sitzelements (30) ausgerichtet ist.

12. Sitzmöbelstück (100) nach einem der vorstehenden Ansprüche mit dem folgenden zusätzlichen Merkmal:
a. das Sitzmöbelstück weist ein Rückenlehnenelement (90) mit einer Rückenlehnenfläche (92) auf, welches um einen Rückenlehnenachse (R) schwenkbeweglich an der Basis (10) oder am Sitzelement (30) angebracht ist.
vorzugsweise mit den zusätzlichen Merkmalen:
b. das Sitzmöbelstück weist einen weiteren Elektromotor auf, mittels dessen das Rücklehnenelement (90) um die Rückenlehnenachse (R) schwenkbar ist, und/oder
c. das Sitzmöbelstück weist beidseitig des Sitzelements Armlehnenflächen (94) auf, die gemeinsam mit dem Rückenlehnenelement (90) um die Rückenlehnenachse (R) verschwenkbar sind.

13. Sitzmöbelstück (100) nach einem der vorstehenden Ansprüche mit dem folgenden zusätzlichen Merkmal:
a. die Basis (10) weist einen Aufstellabschnitt (12A) sowie einen verlagerbaren Abschnitt (12B) auf, und
b. zwischen dem Aufstellabschnitt (12A) oder einem demgegenüber drehbaren Zwischenabschnitt (12C) einerseits und dem verlagerbaren Abschnitt (12B) andererseits ist ein Aufstehhilfen-Getriebe (60) vorgesehen, durch das der verlagerbare Abschnitt (12B) zwischen einer Nutzposition und einer Aufstehposition verlagerbar ist, und
c. das Aufstehhilfen-Getriebe (60) weist zwei Getriebelaschen (62, 64) auf, die sich vom verlagerbaren Abschnitt (12B) in Möbellängsrichtung nach hinten erstrecken,
vorzugsweise mit dem zusätzlichen Merkmal:
d. die beiden Getriebelaschen (62, 64) erstrecken sich ausgehend vom Aufstellabschnitt (12A) oder dem Zwischenabschnitt (12C) in der Nutzposition des verlagerbaren Abschnitts (12B) schräg nach oben und in der Aufstehposition schräg nach unten.

14. Sitzmöbelstück (100) nach einem der vorstehenden Ansprüche mit mindestens einem der folgenden zusätzlichen Merkmale:
a. das Sitzmöbelstück weist einen ersten Elektromotor, der auf das Laschengetriebe wirkt, und einen zweiten Elektromotor auf, der auf das Kippgetriebe wirkt, oder das Sitzmöbelstück (100) weist einen gemeinsamen Elektromotor (80) auf, der auf das Kippgetriebe (20) und auf das Laschengetriebe (40) wirkt, und/oder
b. der Elektromotor (80) ist als längenveränderlicher Linearmotor ausgebildet, der mit einem Ende um eine erste Motorenschwenkachse (M1) schwenkbeweglich an einer Tragstruktur (34) des Sitzelements (30) angelenkt ist und der mit einem zweiten Ende um eine zweite Motorenschwenkachse (M2) schwenkbeweglich am Steuerglied (82) angelenkt ist, und/oder
c. die Tragstruktur (34) des Sitzelements (30) weist zwei zueinander parallele Wangen (34A, 34B) auf, die durch einen Zapfen (37) verbunden oder durchdrungen sind, der die Hauptachse (H) zum Verkippen des Sitzelements (30) gegenüber der Basis (10) definiert, und/oder
d. der Elektromotor (80) ist in einem Zwischenraum (34C) zwischen den Wangen (34A, 34B) der Tragstruktur (34) des Sitzelements (30) angeordnet oder der Elektromotor (80) ist exzentrisch und außerhalb des durch die Wangen (34A, 34B) gebildeten Zwischenraums (34C) angeordnet, und/oder
e. mindestens eine Schwenklasche (46, 68) ist an einer Wange (34A, 34B) der Tragstruktur des Sitzelements (30) und/oder des Beinauflageelements (70) schwenkbar angebracht, wobei ein beabstandender Achsabschnitt vorgesehen ist, sodass in Richtung der Schwenkachse mindestens ein Freiraum von 10 mm zwischen der Schwenklasche und der Wange vorgesehen ist.

## Claims

1. Piece of seating furniture (100) having the following features:
a. the piece of seating furniture (100) has a base (10) for setting up in a fixed position, and
b. the piece of seating furniture (100) has a seating element (30) which is tiltable in relation to the base (10) and has a seating surface (32) and a carrying structure (34), and
c. the piece of seating furniture (100) has a leg support element (70) which is movable in relation to the seating element (30) and has a leg support surface (72) and a carrying structure (74), and
d. the leg support element (70) is connected to the seating element (30) by means of a link mechanism (40), wherein the leg support element (70) is movable between a storage position under the seating element (30) and a use position in front of the seating element (30) by means of the link mechanism (40),
**characterized by** the following additional features:
e. the piece of seating furniture has an electric motor (80) below the seating surface (32), the electric motor acting on the link mechanism (40) via a push rod (86), and
f. to connect the leg support element (70) to the seating element (30), the link mechanism (40) has at least one pivoting link (48) which, during the transfer of the leg support element (70) from the storage position into the use position, is arranged sometimes in a slot (78) partially penetrating the leg support surface (72).

2. Piece of seating furniture (100) according to Claim 1 having the following additional features:
a. a tilting mechanism (20) which can be driven by means of the electric motor (80) is provided for controlling a tilting movement of the seating element (30), and
b. the electric motor (80) acts on the tilting mechanism (20) and the link mechanism (40) by means of a common control member (82),
preferably having the additional features:
c. the tilting mechanism (20) is designed to bring about a shifting of the seating element (30) in relation to the base (10) during the movement of the control member (82) from a starting position into an end position, wherein the tilting mechanism (20) is furthermore designed in such a manner that, in at least one movement phase of the control member (82), the relative position of the seating element (30) with respect to the base (10) remains unchanged, and/or
d. the link mechanism (40) is designed to bring about a shifting of the leg support element (70) in relation to the seating element (30) during the movement of the control member (82) from a starting position into an end position, wherein the link mechanism (40) is furthermore designed in such a manner that, in at least one movement phase of the control member (82), the relative position of the leg support element (70) with respect to the seating element (30) remains unchanged.

3. Piece of seating furniture (100) according to one of the preceding claims having the following additional feature:
a. the movement of the seating element (30) in relation to the base (10) is coupled to the movement of the leg support element (70) in relation to the seating element (30) in such a manner that
i. in a first movement phase, the leg support element (70) is transferred from the storage position into the use position, and
ii. in a subsequent second movement phase, the seating element (30) is tilted together with the leg support element (70) in relation to the base (10) while the leg support element (70) remains in a fixed position with respect to the seating element (30).

4. Piece of seating furniture (100) according to Claim 3 having the following additional feature:
a. in a position toward the end of the first movement phase and before the beginning of the second movement phase, the leg support element (70) is oriented in such a manner that the leg support surface (72) drops forward,
preferably having the additional feature:
b. in a position toward the end of the second movement phase, the leg support element (70) is oriented in such a manner that the leg support surface (72) is oriented horizontally or drops rearward.

5. Piece of seating furniture (100) according to one of the preceding claims having the following additional feature:
a. the link mechanism (40) has at least one front and a rear pivoting link (48, 46) which are each attached at one end to a seating-element-side connecting portion (35) so as to be pivotable about first and second pivot axes (L1, L2) differing from each other and are each attached at the other end to a leg-support-element-side connecting portion (75) so as to be pivotable about third and fourth pivot axes (L3, L4) differing from each other,
preferably having the additional features:
b. the seating-element-side connecting portion (35) is formed by a part of the carrying structure (34) of the seating element (30), said carrying structure being provided in a fixed position with respect to the seating surface (32), and/or
c. the leg-support-element-side connecting portion (75) is formed by a part of the carrying structure (74) of the leg support element (70), said carrying structure being provided in a fixed position with respect to the leg support surface (72).

6. Piece of seating furniture (100) according to Claim 5 having the following additional feature:
a. the push rod (86) via which the electric motor acts on the link mechanism (40) is connected to the link mechanism (40) in such a manner that it acts on the front pivoting link (48) directly or via an additional push link (88), wherein the rear pivoting link (46) is indirectly moved at the same time because of the movement of the front pivoting link (48),
preferably having the additional features:
b. the push rod (86) is guided displaceably on the carrying structure (34) of the seating element (30), and
c. the push link (88) is attached to the push rod (86) so as to be pivotable about a first push link axis (S1) and the push link (88) is attached to the front pivoting link (48) so as to be pivotable about a second push link axis (S2).

7. Piece of seating furniture (100) according to one of the preceding claims having the following additional feature:
a. the push rod (86) is guided by means of a slotted guide mechanism with a slotted guide track (36) and a slotted guide slider (87) which is movable along the slotted guide track (36),
preferably having the additional feature:
b. the slotted guide track (36) has a slotted guide track portion (36A), the shaping of which is configured in such a manner that the first push link axis (S1) describes a circular path in relation to the carrying structure (34) of the seating element (30) and in relation to the second push link axis (S2) .

8. Piece of seating furniture (100) according to one of Claims 5 to 7 having the following additional features:
a. the link mechanism (40) has a securing strut (50) which is attached to the rear pivoting link (46) so as to be pivotable about a fifth pivot axis (L6) and is attached to the front pivoting link (48) so as to be pivotable about a sixth pivot axis (L5), and
b. a connecting line between the first and the third pivot axis (L1, L3) encloses an angle of at least 15° with a connecting line between the first and the fifth pivot axis (L1, L5), and
c. a connecting line between the second and the fourth pivot axis (L2, L4) encloses an angle of at least 15° with a connecting line between the second and the sixth pivot axis (L2, L6),
preferably having the additional feature:
d. the securing strut (50) is attached to the rear pivoting link (46) or to the front pivoting link (48) so as to be pivotable and displaceable to a limited extent such that it forces there to be a minimum distance between the fifth and the sixth pivot axis (L5, L6).

9. Piece of seating furniture (100) according to one of the preceding claims having the following additional features:
a. the tilting mechanism (20) has a main axis (H) which is fixed in position with respect to the seating element (30) and with respect to the base (10) and about which the seating element (30) is tiltable in relation to the base (10), and
b. the tilting mechanism (20) is assigned a control member (82) which can be pivoted about a first control member axis (G1) by the electric motor (80) and on which a slotted guide slider (83) is provided which, over the course of the pivoting of the control member (82), is movable along a slotted guide track (16) such that the seating element (30) is thereby tilted about the main axis (H),
preferably having the additional feature:
a. the slotted guide track (16) has a slotted guide track portion (16A) which has the shape of a circular portion with respect to the first control member axis (G1).

10. Piece of seating furniture (100) according to Claim 9 having the following additional features:
a. the slotted guide track (16) is provided on a slotted guide element (14), which is provided in a tiltproof manner on the base (10), and
b. the control member axis (G1) is provided in a fixed position with respect to the seating element (30).

11. Piece of seating furniture (100) according to one of the preceding claims having the following additional feature:
a. the electric motor (80) is in the form of a linear motor with an extendable rotor (81), wherein the rotor (81) is oriented in a main plane of extent (+/- 10°) of the seating element (30).

12. Piece of seating furniture (100) according to one of the preceding claims having the following additional feature:
a. the piece of seating furniture has a backrest element (90) with a backrest surface (92), said backrest element being attached to the base (10) or to the seating element (30) so as to be pivotable about a backrest axis (R),
preferably having the additional features:
b. the piece of seating furniture has a further electric motor by means of which the backrest element (90) is pivotable about the backrest axis (R), and/or
c. the piece of seating furniture has, on both sides of the seating element, armrests (94) which are pivotable together with the backrest element (90) about the backrest axis (R) .

13. Piece of seating furniture (100) according to one of the preceding claims having the following additional feature:
a. the base (10) has a setting-up portion (12A) and a shiftable portion (12B), and
b. a standing-up aid mechanism (60) is provided between the setting-up portion (12A) or an intermediate portion (12C) which is rotatable in relation thereto, on the one hand, and the shiftable portion (12B), on the other hand, by means of which standing-up aid mechanism the shiftable portion (12B) is shiftable between a use position and a standing-up position, and
c. the standing-up aid mechanism (60) has two mechanism links (62, 64) which extend rearward from the shiftable portion (12B) in the longitudinal direction of the furniture,
preferably having the additional feature:
d. the two mechanism links (62, 64) extend from the setting-up portion (12A) or the intermediate portion (12C) obliquely upward in the use position of the shiftable portion (12B) and obliquely downward in the standing-up position.

14. Piece of seating furniture (100) according to one of the preceding claims having at least one of the following additional features:
a. the piece of seating furniture has a first electric motor which acts on the link mechanism, and a second electric motor which acts on the tilting mechanism, or the piece of seating furniture (100) has a common electric motor (80) which acts on the tilting mechanism (20) and on the link mechanism (40), and/or
b. the electric motor (80) is designed as a length-changeable linear motor which is coupled at one end to a carrying structure (34) of the seating element (30) so as to be pivotable about a first motor pivot axis (M1) and is coupled at a second end to the control member (82) so as to be pivotable about a second motor pivot axis (M2), and/or
c. the carrying structure (34) of the seating element (30) has two mutually parallel cheeks (34A, 34B) which are connected or penetrated by a pin (37) which defines the main axis (H) for tilting the seating element (30) in relation to the base (10), and/or
d. the electric motor (80) is arranged in an intermediate space (34C) between the cheeks (34A, 34B) of the carrying structure (34) of the seating element (30), or the electric motor (80) is arranged excentrically and outside the intermediate space (34C) formed by the cheeks (34A, 34B), and/or
e. at least one pivoting link (46, 68) is attached pivotably to a cheek (34A, 34B) of the carrying structure of the seating element (30) and/or of the leg support element (70), wherein a spacing axial portion is provided such that at least one clearance of 10 mm between the pivoting link and the cheek is provided in the direction of the pivot axis.

## Revendications

1. Meuble de type siège (100), comprenant les caractéristiques suivantes :
a. le meuble de type siège (100) dispose d'une base (10) pour sa mise en place fixée en position, et
b. le meuble de type siège (100) dispose d'un élément de siège (30) pouvant basculer par rapport à la base (10), avec une surface de siège (32) et une structure porteuse (34), et
c. le meuble de type siège (100) dispose d'un élément de support des jambes (70) déplaçable par rapport à l'élément de siège (30), avec une surface de support des jambes (72) et une structure porteuse (74), et
d. l'élément de support des jambes (70) est relié à l'élément de siège (30) au moyen d'un mécanisme à branches (40), l'élément de support des jambes (70) pouvant être déplacé au moyen du mécanisme à branches (40) entre une position rangée sous l'élément de siège (30) et une position d'utilisation devant l'élément de siège (30),
**caractérisé par** les caractéristiques supplémentaires suivantes :
e. le meuble de type siège dispose d'un moteur électrique (80) sous la surface du siège (32), lequel agit par le biais d'une bielle de poussée (86) sur le mécanisme à branches (40), et
f. le mécanisme à branches (40), pour la liaison de l'élément de support des jambes (70) à l'élément de siège (30), présente au moins une branche pivotante (48) qui, lors du transfert de l'élément de support des jambes (70) de la position de rangement à la position d'utilisation, est temporairement disposé dans une fente (78) interrompant partiellement la surface de support des jambes (72).

2. Meuble de type siège (100) selon la revendication 1, comprenant les caractéristiques supplémentaires suivantes :
a. un mécanisme de basculement (20) est prévu pour commander un mouvement de basculement de l'élément de siège (30), lequel peut être entraîné au moyen du moteur électrique (80), et
b. le moteur électrique (80) agit au moyen d'un organe de commande commun (82) sur le mécanisme de basculement (20) et le mécanisme à branches (40),
de préférence comprenant les caractéristiques supplémentaires suivantes :
c. le mécanisme de basculement (20) est réalisé de manière à provoquer, lors du déplacement de l'organe de commande (82) d'une position initiale à une position finale, un déplacement de l'élément de siège (30) par rapport à la base (10), le mécanisme de basculement (20) étant en outre réalisé de telle sorte que dans au moins une phase de déplacement de l'organe de commande (82), la position relative de l'élément de siège (30) par rapport à la base (10) reste inchangée, et/ou
d. le mécanisme à branches (40) est réalisé de manière à provoquer, lors du déplacement de l'organe de commande (82) d'une position initiale à une position finale, un décalage de l'élément de support des jambes (70) par rapport à l'élément de siège (30), le mécanisme à branches (40) étant en outre réalisé de telle sorte que dans au moins une phase de déplacement de l'organe de commande (82), la position relative de l'élément de support des jambes (70) par rapport à l'élément de siège (30) reste inchangée.

3. Meuble de type siège (100) selon l'une quelconque des revendications précédentes, comprenant la caractéristique supplémentaire suivante :
a. le déplacement de l'élément de siège (30) par rapport à la base (10) est accouplé au déplacement de l'élément de support des jambes (70) par rapport à l'élément de siège (30) de telle sorte que
i. dans une première phase de déplacement, l'élément de support des jambes (70) soit transféré de la position de rangement à la position d'utilisation, et
ii. dans une deuxième phase de déplacement subséquente, l'élément de siège (30) soit basculé conjointement avec l'élément de support des jambes (70) par rapport à la base (10) tandis que l'élément de support des jambes (70) reste fixé en position par rapport à l'élément de siège (30).

4. Meuble de type siège (100) selon la revendication 3, comprenant la caractéristique supplémentaire suivante :
a. dans une position vers la fin de la première phase de déplacement et avant le début de la deuxième phase de déplacement, l'élément de support des jambes (70) est orienté de telle sorte que la surface de support des jambes (72) descende vers l'avant,
de préférence comprenant la caractéristique suivante :
b. dans une position vers la fin de la deuxième phase de déplacement, l'élément de support des jambes (70) est orienté de telle sorte que la surface de support des jambes (72) soit orientée horizontalement ou descende vers l'arrière.

5. Meuble de type siège (100) selon l'une quelconque des revendications précédentes, comprenant la caractéristique supplémentaire suivante :
a. le mécanisme à branches (40) présente au moins une branche de pivotement avant et une branche de pivotement arrière (48, 46) qui sont montées de manière déplaçable par pivotement à chaque fois d'une part autour de premier et deuxième axes de pivotement s'écartant l'un de l'autre (L1, L2) au niveau d'une portion de liaison du côté de l'élément de siège (35) et à chaque fois d'autre part autour de troisième et quatrième axes de pivotement s'écartant l'un de l'autre (L3, L4) au niveau d'une portion de liaison du côté de l'élément de support des jambes (75),
de préférence comprenant les caractéristiques supplémentaires suivantes :
b. la portion de liaison du côté de l'élément de siège (35) est formée par une partie de la structure porteuse (34) de l'élément de siège (30) qui est prévue de manière fixée en position par rapport à la surface de siège (32), et/ou
c. la portion de liaison du côté de l'élément de support des jambes (75) est formée par une partie de la structure porteuse (74) de l'élément de support des jambes (70) qui est prévue de manière fixée en position par rapport à la surface de support des jambes (72).

6. Meuble de type siège (100) selon la revendication 5, comprenant la caractéristique supplémentaire suivante :
a. la tige de poussée (86) par le biais de laquelle le moteur électrique agit sur le mécanisme à branches (40) est reliée au mécanisme à branches (40) de telle sorte qu'elle agisse directement par le biais d'une branche de poussée supplémentaire (88) sur la branche de pivotement avant (48), la branche de pivotement arrière (46) étant entraînée indirectement du fait du déplacement de la branche de pivotement avant (48),
de préférence comprenant les caractéristiques supplémentaires suivantes :
b. la tige de poussée (86) est guidée de manière déplaçable sur la structure porteuse (34) de l'élément de siège (30), et
c. la branche de poussée (88) est montée de manière déplaçable par pivotement autour d'un premier axe de branche de poussée (S1) au niveau de la tige de poussée (86) et la branche de poussée (88) est montée de manière déplaçable par pivotement autour d'un deuxième axe de branche de poussée (S2) au niveau de la branche de pivotement avant (48).

7. Meuble de type siège (100) selon l'une quelconque des revendications précédentes, comprenant la caractéristique supplémentaire suivante :
a. la tige de poussée (86) est guidée au moyen d'un guide à coulisse avec une piste de coulisse (36) et un coulisseau (87) déplaçable le long de la piste de coulisse (36),
de préférence comprenant la caractéristique supplémentaire suivante :
b. la piste de coulisse (36) présente une portion de piste de coulisse (36A) dont la forme est configurée de telle sorte que le premier axe de branche de poussée (S1) décrive une trajectoire circulaire par rapport à la structure porteuse (34) de l'élément de siège (30) et par rapport au deuxième axe de branche de poussée (S2).

8. Meuble de type siège (100) selon l'une quelconque des revendications 5 à 7, comprenant les caractéristiques supplémentaires suivantes :
a. le mécanisme à branches (40) présente un montant de fixation (50) qui est monté de manière à pouvoir pivoter autour d'un cinquième axe de pivotement (L5) au niveau de la branche de pivotement arrière (46) et de manière à pouvoir pivoter autour d'un sixième axe de pivotement (L6) au niveau de la branche de pivotement avant (48), et
b. une ligne de liaison entre le premier et le troisième axe de pivotement (L1, L3) forme avec la ligne de liaison entre le premier et le cinquième axe de pivotement (L1, L5) un angle d'au moins 15°, et
c. une ligne de liaison entre le deuxième et le quatrième axe de pivotement (L2, L4) forme avec une ligne de liaison entre le deuxième et le sixième axe de pivotement (L2, L6) un angle d'au moins 15°,
de préférence comprenant la caractéristique supplémentaire suivante :
d. le montant de fixation (50) est monté au niveau de la branche de pivotement arrière (46) de manière pivotante et de manière déplaçable dans une mesure limitée au niveau de la branche de pivotement avant (48) de telle sorte qu'il force une distance minimale entre le cinquième et le sixième axe de pivotement (L5, L6).

9. Meuble de type siège (100) selon l'une quelconque des revendications précédentes, comprenant les caractéristiques supplémentaires suivantes :
a. le mécanisme de basculement (20) présente un axe principal (H) fixé par rapport à l'élément de siège (30) et par rapport à la base (10), autour duquel l'élément de siège (30) peut basculer par rapport à la base (10), et
b. au mécanisme de basculement (20) est associé un organe de commande (82) pouvant être pivoté par un moteur électrique (80) autour d'un premier axe d'organe de commande (G1), au niveau duquel organe de commande est prévu un coulisseau (83 déplaçable au cours du pivotement de l'organe de commande (82) le long d'une piste de coulisse (16) de telle sorte que l'élément de siège (30) soit de ce fait basculé autour de l'axe principal (H),
de préférence comprenant la caractéristique supplémentaire suivante :
a. la piste de coulisse (16) présente une portion de piste de coulisse (16A) qui présente la forme d'une portion de cercle par rapport au premier axe d'organe de commande (G1).

10. Meuble de type siège (100) selon la revendication 9, comprenant les caractéristiques supplémentaires suivantes :
a. la piste de coulisse (16) est prévue au niveau d'un élément de coulisse (14) fixé à la base (10) de manière non pivotante, et
b. l'axe d'organe de commande (G1) est prévu de manière fixée en position par rapport à l'élément de siège (30).

11. Meuble de type siège (100) selon l'une quelconque des revendications précédentes, comprenant la caractéristique supplémentaire suivante :
a. le moteur électrique (80) est réalisé sous forme de moteur linéaire avec un rotor (81) pouvant être sorti, le rotor (81) étant orienté dans un plan d'étendue principale (+/- 10°) de l'élément de siège (30).

12. Meuble de type siège (100) selon l'une quelconque des revendications précédentes, comprenant la caractéristique supplémentaire suivante :
a. le meuble de type siège présente un élément de dossier (90) avec une surface de dossier (92), lequel est monté de manière à pouvoir pivoter autour d'un axe de dossier (R) sur la base (10) ou sur l'élément de siège (30),
de préférence comprenant les caractéristiques supplémentaires suivantes :
b. le meuble de type siège présente un moteur électrique supplémentaire au moyen duquel l'élément de dossier (90) peut pivoter autour de l'axe de dossier (R), et/ou
c. le meuble de type siège présente des deux côtés de l'élément de siège des surfaces d'accoudoirs (94) qui peuvent pivoter conjointement avec l'élément de dossier (90) autour de l'axe de dossier (R).

13. Meuble de type siège (100) selon l'une quelconque des revendications précédentes, comprenant la caractéristique supplémentaire suivante :
a. la base (10) présente une portion de mise en place (12A) et une portion déplaçable (12B), et
b. entre la portion de mise en place (12A) ou une portion intermédiaire (12C) pouvant tourner par rapport à celle-ci d'une part et la portion déplaçable (12B) d'autre part, est prévu un mécanisme d'aide au levage (60) par le biais duquel la portion déplaçable (12B) peut être déplacée entre une position d'utilisation et une position de levage, et
c. le mécanisme d'aide au levage (60) présente deux branches de mécanisme (62, 64) qui s'étendent vers l'arrière depuis la portion déplaçable (12B) dans la direction longitudinale du siège,
de préférence comprenant la caractéristique supplémentaire suivante :
d. les deux branches de mécanisme (62, 64) s'étendent à partir de la portion de mise en place (12A) ou de la portion intermédiaire (12C) dans la position d'utilisation de la portion déplaçable (12b) obliquement vers le haut et dans la position de levage obliquement vers le bas.

14. Meuble de type siège (100) selon l'une quelconque des revendications précédentes, comprenant au moins l'une des caractéristiques supplémentaires suivantes :
a. le meuble de type siège présente un premier moteur électrique qui agit sur le mécanisme à branches et un deuxième moteur électrique qui agit sur le mécanisme de basculement ou le meuble de type siège (100) présente un moteur électrique commun (80) qui agit sur le mécanisme de basculement (20) et sur le mécanisme à branches (40), et/ou
b. le moteur électrique (80) est réalisé sous forme de moteur linéaire de longueur variable qui est articulé par une extrémité à une structure porteuse (34) de manière à pouvoir pivoter autour d'un premier axe de pivotement de moteur (M1) de l'élément de siège (30) et par une deuxième extrémité à l'organe de commande (82) de manière à pouvoir pivoter autour d'un deuxième axe de pivotement de moteur (M2), et/ou
c. la structure porteuse (34) de l'élément de siège (30) présente deux parois parallèles l'une à l'autre (34A, 34B) qui sont connectées par un tourillon (37) ou traversées par ce dernier, lequel définit l'axe principal (H) pour le basculement de l'élément de siège (30) par rapport à la base (10), et/ou
d. le moteur électrique (80) est disposé dans un espace intermédiaire (34C) entre les parois (34A, 34B) de la structure porteuse (34) de l'élément de siège (30) ou le moteur électrique (80) est disposé de manière excentrée et à l'extérieur de l'espace intermédiaire (34C) formé par les parois (34A, 34B), et/ou
e. au moins une branche de pivotement (46, 68) est montée de manière à pouvoir pivoter au niveau d'une paroi (34A, 34B) de la structure porteuse de l'élément de siège (30) et/ou de l'élément de support des jambes (70), une portion d'axe espacée étant prévue de telle sorte que dans la direction de l'axe de pivotement, au moins un espace libre de 10 mm soit prévu entre la branche de pivotement et la paroi.
